(19) Europäisches Patentamt
European Patent Office
Office européen des brevets

(11) Veröffentlichungsnummer: 0 148 381
B1

# (12) EUROPÄISCHE PATENTSCHRIFT

(45) Veröffentlichungstag der Patentschrift: 31.08.88

(21) Anmeldenummer: 84114103.9

(22) Anmeldetag: 22.11.84

(51) Int. Cl.⁴: C 08 G 18/80, C 08 G 18/50, C 08 G 18/32, C 08 G 18/10, C 08 G 18/78, C 07 C 127/19

**ERRATUM**

| DIE TEXTSTELLE:<br>TEXT PUBLISHED:<br>LE PASSAGE SUIVANT: | (SEITE, SPALTE, ZEILE)<br>(PAGE, COLUMN, LINE)<br>(PAGE, COLONNE, LIGNE) | | LAUTET BERICHTIGT:<br>SHOULD READ:<br>DEVRAIT ETRE LU: |
|---|---|---|---|
| (1200 Upm) 42,3 eines | 7 | 6 | (1200 Upm) 42,3 Teile eines |

Tag der Entscheidung über die Berichtigung / Date of decision on rectification / Date de décision portant sur modification: 10.01.89

Ausgabe- und Veröffentlichungstag / Issue and publication date / Date d'edition et de publication: 28.06.89

Patbl.Nr / EPB no / Bull. no: 89/26

(19) Europäisches Patentamt
European Patent Office
Office européen des brevets

(11) Veröffentlichungsnummer: **0 148 381**
**B1**

# (12) EUROPÄISCHE PATENTSCHRIFT

(45) Veröffentlichungstag der Patentschrift: **31.08.88**

(51) Int. Cl.⁴: **C 08 G 18/80,** C 08 G 18/50, C 08 G 18/32, C 08 G 18/10, C 08 G 18/78, C 07 C 127/19

(21) Anmeldenummer: **84114103.9**

(22) Anmeldetag: **22.11.84**

(54) **Flüssige, Harnstoffgruppen enthaltende Polyisocyanatmischungen, Verfahren zu deren Herstellung und deren Verwendung zur Herstellung von kompakten oder zelligen Polyurethan- und/oder Polyisocyanurat-Kunststoffen insbesondere Polyurethan-Weichschaumstoffen.**

(30) Priorität: **26.11.83 DE 3342864**

(43) Veröffentlichungstag der Anmeldung: **17.07.85 Patentblatt 85/29**

(45) Bekanntmachung des Hinweises auf die Patenterteilung: **31.08.88 Patentblatt 88/35**

(84) Benannte Vertragsstaaten: **BE DE FR GB IT NL SE**

(56) Entgegenhaltungen:
**EP-A-0 033 458**
**EP-A-0 093 336**
**FR-A-1 513 225**
**FR-A-2 266 712**

(73) Patentinhaber: **BASF Aktiengesellschaft, Carl-Bosch- Strasse 38, D-6700 Ludwigshafen (DE)**

(72) Erfinder: **Buethe, Ingolf, Dr., Am Wasserturm 1, D-6737 Boehl- Iggelheim (DE)**
Erfinder: **Marx, Matthias, Dr., Seebacher Strasse 49, D-6702 Bad Duerkheim (DE)**
Erfinder: **Schoenleben, Willibald, Dr., Blumenthalstrasse 41, D-6900 Heidelberg (DE)**

**EP 0 148 381 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Urethan-, Allophanat-, Biuret-, Harnstoff-, Carbodiimid- und/oder Isocyanuratgruppen enthaltende organische Polyisocyanate, die üblicherweise in unmodifizierten Polyisocyanaten der gleichen Art gelöst sind, sind Gegenstand zahlreicher Patente und -anmeldungen. Beispielhaft genannt seien Urethangruppen aufweisende Polyisocyanate gemäß DE-A-2 513 793 (GB-PS-1 450 660) und DE-A-2 513 796 (GB-PS-1 444 192), Allophanatgruppen aufweisende Polyisocyanate gemäß GB-PS-994 890, Biuretgruppen aufweisende Polyisocyanate gemäß DE-A-1 215 365 (US-PS-3 441 588), Harnstoffgruppen aufweisende Polyisocyanate gemäß DE-A-1 008 484 (GB-PS-791 852), Carbodiimidgruppen aufweisende Polyisocyanate gemäß EP-A-57 862 und Isocyanuratgruppen aufweisende Polyisocyanate gemäß DE-PS-2 616 416 (GB-PS-1 571 933).

Auf diese Weise können bei Raumtemperatur kristalline Polyisocyanate zur Verbesserung der Verarbeitbarkeit verflüssigt werden oder bei flüssigen Polyisocyanaten mit hohem Dampfdruck kann durch eine Erhöhung des Molekulargewichts die Flüchtigkeit vermindert werden.

Ein Verfahren zur Herstellung von modifizierten Polyisocyanaten durch Umsetzung von organischen Polyisocyanaten mit unterschüssigen Mengen an Polyaminen oder Polyalkylen-polyaminen mit Molekulargewichten zwischen 146 und 300, die nicht mehr als 3 nichtaromatisch gebundene, basische Stickstoffatome aufweisen, von denen mindestens 2 primäre und/oder sekundäre Aminstickstoffatome darstellen, wird beschrieben in der EP-A-0 033 458. Mit Hilfe der thermisch problemlosen Reaktion, die ohne Mitverwendung von Verdünnungsmitteln und/oder den Einsatz von technisch aufwendigen Vorrichtungen zur Beherrschung der Reaktionswärme durchgeführt werden kann, lassen sich bei Raumtemperatur flüssige Polyisocyanate und deren Gemische leicht in situ zu Suspensionen von Polyharnstoffen in diesen Polyisocyanaten modifizieren. Die Produkte besitzen gegenüber den Polyisocyanaten eine erhöhte Viskosität und bisweilen auch thixotrope Eigenschaften.

Nach Angaben der Europäischen Patentschriften Nrs. 4617, 4618 und 4879 können Polyurethan-Weichschaumstoffe mit hoher Trag- und Stoßabsorptionsfähigkeit bzw. Schwerentflammbarkeit aus Mischungen aus Diphenylmethan-diisocyanaten und Polyphenyl-polymethylen-polyisocyanaten - im folgenden kurz Roh-MDI genannt - mit einem hohen Gehalt an Diphenylmethan-diisocyanaten im folgenden kurz MDI genannt - oder Urethangruppen enthaltendem Roh-MDI hergestellt werden. Nachteilig an diesen Verfahren ist, daß die Alterungsbeständigkeit derartiger Polyurethan-Weichschaumstoffe bei erhöhter Temperatur und Luftfeuchtigkeit, wie sie beispielsweise für Polstermaterialien im Automobilbau gefordert werden, vielfach unzureichend erfüllt wird.

Die Aufgabe der vorliegenden Erfindung bestand darin, Polyurethan-Weichschaumstoffe mit guter Alterungsbeständigkeit bei hohen Temperaturen und Luftfeuchtigkeit herzustellen.

Überraschenderweise konnte diese Aufgabe durch die Verwendung von mit ausgewählten Polyaminen modifizierten, Harnstoffgruppen enthaltenden Polyisocyanatmischungen als Aufbaukomponente für die Isocyanatpolyaddition gelöst werden.

Gegenstand der Erfindung sind somit bei Raumtemperatur flüssige, Harnstoffgruppen enthaltende Polyisocyanatmischungen mit einem NCO-Gehalt von 15 bis 30 Gew.-%, vorzugsweise 20 bis 28 Gew.-%, und einem Gehalt an Diphenylmethandiisocyanat-Isomeren von 55 bis 90 Gew.-%, vorzugsweise 60 bis 85 Gew.-%, wobei die Gew.-% bezogen sind auf das Gesamtgewicht der Polyisocyanatmischung, und die erhalten werden durch partielle Umsetzung von

A) Polyoxyalkylen-polyaminen mit einer Funktionalität von 2 bis 5, vorzugsweise 2,1 bis 3 und einer Aminzahl von 20 bis 250, vorzugsweise von 20 bis 100 mit

B1) Roh-MDI mit einem Gehalt an MDI von 55 bis 90 Gew.-%, vorzugsweise 60 bis 85 Gew.-%, bezogen auf das Roh-MDI-Gewicht oder

B2) mindestens einem Diphenylmethan-diisocyanat-Isomeren und anschließendem Verdünnen des Harnstoffgruppen enthaltenden Reaktionsprodukts mit Roh-MBI mit einem MDI-Gehalt von 30 bis 85 Gew.-%, vorzugsweise 40 bis 60 Gew.%, bezogen auf das Gesamtgewicht.

Gegenstand der Erfindung sind ferner bei Raumtemperatur flüssige, Harnstoffgruppen enthaltende Diphenylmethan-diisocyanatmischungen mit einem NCO-Gehalt von 15 bis 28 Gew.-% gemäß Anspruch 2 und Verfahren zur Herstellung der erfindungsgemäßen, bei Raumtemperatur flüssigen, Harnstoffgruppen enthaltenden Polyisocyanatmischungen, wie sie in Anspruch 5 beschrieben werden, sowie die Verwendung der erfindungsgemäßen Produkte zur Herstellung von kompakten oder zelligen Polyurethan- und/oder Polyisocyanurat-Kunststoffen, insbesondere Polyurethan-Weichschaumstoffen.

Zu den für die erfindungsgemäßen Polyisocyanatmischungen verwendbaren Ausgangskomponenten sowie zu den Aufbaukomponenten zur Herstellung der Polyurethan- und/oder Polyisocyanurat-Kunststoffe ist folgendes auszuführen:

Als Polyoxyalkylen-polyamine (A) kommen lineare und/oder verzweigte, d. h. 2- bis 5-funktionelle, vorzugsweise 2,1- bis 3-funktionelle Polyoxyalkylen-polyamine mit Aminzahlen von 20 bis 250, vorzugsweise von 20 bis 100 in Betracht.

Besonders bewährt haben sich und daher vorzugsweise verwendet werden Polyoxyalkylen-polyamine (A) der allgemeinen Formeln

$$H_2N-(R^2O)_x-R^1-NH_2 \qquad (I),$$

$$H_2N-R^1-(OR^2)_x-NH-(R^2O)_y-R^1-NH_2 \qquad (II),$$

$$H_2N-R^1-(OR^2)_x-T\begin{cases}(R^2O)_y-R^1-NH_2\\(R^2O)_z-R^1-NH_2\end{cases} \qquad (III)$$

und/oder

$$\left.\begin{matrix}H_2N-R^1-(OR^2)_y\\H_2N-R^1-(OR^2)_z\end{matrix}\right\rangle T-(R^2O)_x-R^1-NH-R^1-(OR^2)_{x'}-T\begin{cases}(R^2O)_{y'}-R^1-NH_2\\(R^2O)_{z'}-R^1-NH_2\end{cases} \qquad (IV)$$

in denen bedeuten:

$R^1$ und $R^2$ gleiche oder verschiedene, gegebenenfalls substituierte Alkylenreste mit 2 bis 10, vorzugsweise 2 bis 3 Kohlenstoffatomen, wobei die Alkylenreste $R^2$ der Alkoxygruppen ebenfalls gleich oder verschieden sein können,

T ein von reaktiven Wasserstoffatomen befreit er Rest eines trifunktionellen Startermoleküls für die Alkylenoxid-polymerisation und

x, x', y, y', z und z' gleiche oder verschiedene ganze Zahlen größer als null, so daß die Summe eine Aminzahl von 20 bis 250 ergibt.

Die genannten Polyoxyalkylen-polyamine können einzeln oder als Mischungen eingesetzt werden. Vorzugsweise Anwendung finden Mischungen aus Polyoxyalkylen-polyaminen der Formeln (I) und (II) und solche der Formeln (III) und (IV), wobei die Mischungsverhältnisse von (I) : (II) bzw. (III) : (IV) in den Bereichen von 99,5 : 0,5 bis 20 : 80, vorzugsweise 99,5 : 0,5 bis 50 : 50 liegen.

Die erfindungsgemäß verwendbaren Polyoxyalkylen-polyamine können nach bekannten Verfahren hergestellt werden.

Beispielhaft genannt seien die Cyanoalkylierung von Polyoxyalkylen-polyolen und anschließende Hydrierung des gebildeten Nitrils (US-3 267 050) oder die Aminierung von Polyoxyalkylen-polyolen mit Aminen oder Ammoniak in Gegenwart von Wasserstoff und Katalysatoren (DE-1 215 373).

Geeignete Polyoxyalkylen-polyole können ihrerseits erhalten werden durch Addition von einem oder mehreren Alkylenoxiden mit 2 bis 10 Kohlenstoffatomen, vorzugsweise 2 bis 3 Kohlenstoffatomen, im Alkylenrest, wie z. B. Tetrahydrofuran, 1,3-Propylenoxid, 1,2- bzw. 2,3-Butylenoxid, Styroloxid und vorzugsweise Ethylenoxid und 1,2-Propylenoxid an ein Startermolekül, das 2 bis 5, vorzugsweise 2 bis 3 reaktive Wasserstof fatome gebunden enthält.

Als Startermoleküle kommen vorzugsweise in Betracht: Wasser, Ammoniak, Alkanolamine, wie z. B. Ethanolamin, Diethanolamin, N-Methyl- und N-Ethyl-ethanolamin, N-Methyl- und N-Ethyl-diethanolamin und Triethanolamin, und insbesondere mehrwertige, vorzugsweise zwei- und/oder dreiwertige Alkohole, wie z. B. Ethylen-, 1,2- und 1,3-Propylen-, Diethylen-, Dipropylen-, 1,4-Butylen-, 1,6-Hexamethylen-glykol, Glycerin, Trimethylolpropan und Pentaerythrit.

Zur Herstellung der erfindungsgemäßen Polyisocyanatmischungen eignen sich Roh-MDI's mit einem Gehalt an MDI-Isomeren von 30 bis 90 Gew.-%, bezogen auf das Gesamtgewicht, wobei Roh-MDI mit einem MDI-Gehalt von 55 bis 90 Gew.-%, vorzugsweise 60 bis 85 Gew.-%, nach der Verfahrensvariante B1 direkt mit den Polyoxyalkylen-polyaminen (A) umgesetzt werden kann, während Roh-MDI mit einem MDI-Gehalt von 30 bis 85 Gew.-%, vorzugsweise 40 bis 60 Gew.-%, üblicherweise zum Verdünnen des Harnstoffgruppen enthaltenden MDI nach der Verfahrensvariante B2 oder gegebenenfalls auch von Harnstoffgruppen enthaltendem Roh-MDI mit einem hohen MDI-Gehalt verwendet wird.

Als MDI-Isomere eignen sich 4,4'-, 2,4'- und 2,2'-MDI, wobei Mischungen aus mindestens zwei dieser Isomeren, insbesondere dem 4,4'- und 2,4'-MDI, bevorzugt sind, da sie im Gegensatz zu den im wesentlichen reinen Verbindungen bei Raumtemperatur flüssig sind oder eine bessere Fließfähigkeit besitzen. Bewährt haben sich beispielsweise MDI-Mischungen, die bestehen aus

40 bis 98 Gew.-%, vorzugsweise 50 bis 96 Gew.-% und insbesondere 60 bis 85 Gew.-% 4,4'-MDI,
60 bis 2 Gew.-%, vorzugsweise 50 bis 2 Gew.-% und insbesondere 40 bis 14 Gew.-% 2,4'-MDI und
0 bis 2 Gew.-%, vorzugsweise 0 bis 1 Gew.-% 2,2'-MDI.

Zur Herstellung der erfindungsgemäßen Harnstoffgruppen enthaltenden Polyisocyanatmischungen werden die Polyoxyalkylen-polyamine und Roh-MDI oder MDI-Isomere in solchen Mengen zur Reaktion gebracht, daß das NCO- : NH2-Gruppenverhältnis 1 : 0,005 bis 1 : 0,35, vorzugsweise 1 : 0,01 bis 1 : 0,2 beträgt.

Das Roh-MDI mit einem MDI-Gehalt von 55 bis 90 Gew.-% und das Polyoxyalkylen-polyamin (A) wird nach der Verfahrensvariante B1 bei Temperaturen von 10 bis 120°C, vorzugsweise von 60 bis 90°C unter starker Turbulenz, beispielsweise unter Rühren mit Rührerdrehzahlen von 200 bis 1500 pro Minute, gemischt und gleichzeitig umgesetzt. Nach einer Reaktionszeit von 0,25 bis 60 Minuten, vorzugsweise 5 bis 15 Minuten läßt man die Reaktionsmischung abkühlen.

Werden die erfindungsgemäßen Polyisocyanatmischungen vorwiegend zur Herstellung von Polyurethan-Weichschaumstoffen verwendet, so wird insbesondere nach der Verfahrensvariante B2 verfahren. Hierzu werden das MDI oder vorzugsweise die MDI-Isomerenmischung und die Polyoxyalkylen-polyamine (A) analog B1 bei Temperaturen von 10 bis 120°C, vorzugsweise 60 bis 90°C gemischt und zur Reaktion gebracht. Nach einer Reaktionszeit von 0,25 bis 60 Minuten, vorzugsweise 0,25 bis 15 Minuten, läßt man die Harnstoffgruppen enthaltende Reaktionsmischung auf Temperaturen von 20 bis 80°C abkühlen und verdünnt sie mit Roh-MDI mit einem MDI-Gehalt von 30 bis 85 Gew.-% bis zu dem erfindungsgemäßen NCO-Gehalt von 15 bis 30 Gew.-%. Bei Verwendung von 40 bis 90 Gew.-Teilen eines Harnstoffgruppen enthaltenden MDI oder MDI-Gemisches sind hierfür üblicherweise 60 bis 10 Gew.-Teile-Roh-MDI mit einem MDI-Gehalt zwischen 40 und 65 Gew.-% erforderlich.

Die erfindungsgemäßen flüssigen, Harnstoffgruppen enthaltenden Polyisocyanatmischungen sind bei 15°C mehrere Monate lagerstabil. Sie finden Verwendung zur Herstellung von kompakten oder zelligen Polyurethan- und/oder Polyisocyanurat-Kunststoffen. Flüssige, Harnstoffgruppen enthaltende Diphenylmethan-diisocyanatmischungen nach Anspruch 2 eignen sich ferner zur Modifizierung von organischen Polyisocyanaten.

Da die Polyisocyanatmischungen - wie bereits dargelegt wurde - überraschenderweise die Alterungsbeständigkeit bei hohen Temperaturen und Luftfeuchtigkeit und hierbei insbesondere die Zugfestigkeit von Polyurethan-Weichschaumstoffen mit niedriger Dichte, erheblich verbessern, werden sie vorzugsweise zu deren Herstellung eingesetzt.

Zur Herstellung von Polyurethan-Weichschaumstoffen werden die erfindungsgemäßen flüssigen, Harnstoffgruppen enthaltenden Polyisocyanatmischungen mit Polyhydroxylverbindungen und gegebenenfalls Kettenverlängerungsmitteln in Gegenwart von Katalysatoren, Treibmitteln sowie gegebenenfalls Hilfsmitteln und/oder Zusatzstoffen zur Reaktion gebracht.

Hierzu werden zweckmäßigerweise ausschließlich die erfindungsgemäßen Polyisocyanatmischungen eingesetzt. Dennoch kann es zur Erzielung bestimmter Eigenschaften, beispielsweise zur Verbesserung der Lichtbeständigkeit, Fließfähigkeit, Offenzelligkeit u. a. vorteilhaft sein, die erfindungsgemäßen Polyisocyanatmischungen mit üblichen, gegebenenfalls modifizierten Polyisocyanaten in untergeordneten Mengen, beispielsweise bis zu 40 Gew.-Teilen pro 100 Gew.-Teilen erfindungsgemäßer Polyisocyanatmischung, abzumischen. Hierfür in Betracht kommen beispielsweise aliphatische, cycloaliphatische, arylaliphatische und vorzugsweise aromatische mehrwertige Isocyanate. Im einzelnen seien beispielhaft genannt: aliphatische Diisocyanate, wie Ethylen-, 1,4-Tetramethylen-, 1,6-Hexamethylen- und 1,12-Dodecandiisocyanat; cycloaliphatische Diisocyanate, wie Cyclohexan-1,3- und -1,4-diisocyanat sowie beliebige Gemische dieser Isomeren, 1-Isocyanato-3,3,5-trimethyl-5-isocyanatomethyl-cyclohexan, 2,4- und 2,6-Hexahydrotoluylendiisocyanat sowie beliebige Gemische dieser Isomeren, 4,4'- und 2,4'-Diisocyanato-dicyclohexylmethan; aromatische Diisocyanate, wie 1,3- und 1,4-Phenylen-diisocyanat, 2,4- und 2,6-Toluylen-diisocyanat sowie beliebige Gemische dieser Isomeren und Naphthylen-1,5-diisocyanat. Verwendet werden können ferner modifizierte Polyisocyanate, beispielsweise Carbodiimid-, Allophanat-, Biuret-, Ester-, Isocyanurat- und vorzugsweise Urethangruppen enthaltende Polyisocyanate aus den beispielhaft genannten organischen Polyisocyanaten. Bevorzugt zum Abmischen verwendet werden die technisch gut zugänglichen 2,4- und/oder 2,6-Toluylen-diisocyanate sowie urethangruppenhaltige Polyisocyanate auf Basis von 2,4- und 2,6-Toluylen-diisocyanat, 2,2'-, 2,4'- und 4,4'-MDI und Roh-MDI.

Als Polyhydroxylverbindungen zur Herstellung der Polyurethan-Weichschaumstoffe finden vorzugsweise übliche lineare und/oder verzweigte Polyesterole und insbesondere Polyetherole mit Molekulargewichten von 200 bis 8000, vorzugsweise 800 bis 5000, und insbesondere 1800 bis 3500 Anwendung. In Betracht kommen jedoch auch andere hydroxylgruppenhaltige Polymere mit den obengenannten Molekulargewichten, beispielsweise Polyesteramide, Polyacetale und/oder Polycarbonate, insbesondere solche, hergestellt aus Diphenylcarbonat und Hexandiol-1,6 durch Umesterung.

Die Polyesterole können beispielsweise aus Dicarbonsäuren, vorzugsweise aliphatischen Dicarbonsäuren, mit 2 bis 12, vorzugsweise 4 bis 8 Kohlenstoffatomen im Alkylenrest und mehrwertigen Alkoholen mit 2 bis 10, vorzugsweise 2 bis 6 Kohlenstoffatomen, vorzugsweise Diolen, hergestellt werden. Genannt seien beispielhaft aliphatische Dicarbonsäuren, wie Glutarsäure, Pimelinsäure, Korksäure, Azelainsäure, Sebacinsäure, Undecandisäure, Dodecandisäure und vorzugsweise Adipinsäure und Mischungen aus Bernstein-, Glutar- und Adipinsäure, und aromatische Dicarbonsäuren, wie Phthalsäure und Terephthalsäure. Beispiele für mehrwertige, insbesondere zwei- und dreiwertige Alkohole sind: Ethylenglykol, Diethylenglykol, 1,2- bzw. 1,3-Propylenglykol, Dipropylenglykol, Neopentylglykol, Decandiol-1,10, Glycerin, Trimethylolpropan und vorzugsweise Butandiol-1,4, Hexandiol-1,6 und Mischungen aus Butandiol-1,4, Pentandiol-1,5 und Hexandiol-1,6. Sofern zur Herstellung der Polyesterole mehrwertige, insbesondere dreiwertige Alkohole mitverwendet werden, wird deren Gehalt zweckmäßigerweise so berechnet, daß die Funktionalität der erhaltenen Polyesterole maximal 6, vorzugsweise 2 bis 4 ist.

Die Polyesterole besitzen Molekulargewichte von 500 bis 2 800, vorzugsweise von 1 000 bis 2 000, und

Hydroxylzahlen von 40 bis 280, vorzugsweise von 50 bis 120.

Vorzugsweise als Polyhydroxylverbindungen verwendet werden jedoch Polyetherole, die durch kationische Polymerisation von einem oder mehreren Alkylenoxiden mit 2 bis 4 Kohlenstoffatomen im Alkylenrest oder durch anionische Polymerisation aus Alkylenoxid oder -mischungen und einem Startermolekül, das 2 bis 4, vorzugsweise 2 bis 3 aktive Wasserstoffatome enthält, hergestellt werden.

Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden.

Als Startermoleküle kommen beispielsweise in Betracht: Wasser, organische Dicarbonsäure, wie Bernsteinsäure, Adipinsäure, Phthalsäure und Terephthalsäure, aliphatische und aromatische, gegebenenfalls H-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,4- und 2,6-Toluylendiamin und 4,4'-, 2,4'- und 2,2'-Diamino-diphenylmethan; Monoamine, wie Methylamin, Ethylamin, Isopropylamin, Butylamin, Benzylamin, Anilin, die Toluidine und Naphthylamine. Von den Verbindungen der erwähnten Gruppe sind besonders interessant N,N,N',N'-Tetrakis-(2-hydroxyethyl)-ethylendiamin, N,N,N',N'-Tetrakis-(2-hydroxypropyl)-ethylendiamin, N,N,N',N'',N''-Pentakis-(2-hydroxypropyl-)-ethylentriamin, Phenyldiisopropanolamin und höhere Alkylenoxidaddukte von Anilin.

Als Startermoleküle kommen ferner in Betracht Alkanolamine, wie Ethanolamin, Diethanolamin, N-Methyl- und N-Ethyl-diethanolamin, N-Methyl- und N-Ethyl-dipropanolamin und Triethanolamin, Hydrazin und Hydrazide. Vorzugsweise verwendet werden mehrwertige, insbesondere zwei- und/oder dreiwertige Alkohole, wie Ethylenglykol, Propylenglykol-1,2 und -1,3, Diethylenglykol, Dipropylenglykol, Butylenglykol-1,4, Hexamethylenglykol-1,6, Glycerin, Trimethylol-propan und Pentaerythrit.

Zu den Polyesteramiden zählen z. B. die aus mehrwertigen gesättigten und/oder ungesättigten Carbonsäuren bzw. deren Anhydriden und mehrwertigen gesättigten und/oder ungesättigten Aminoalkoholen, oder Mischungen aus mehrwertigen Alkoholen und Aminoalkoholen und/oder Polyaminen gewonnenen, vorwiegend linearen Kondensate.

Als Polyacetale kommen z. B. die aus Glykolen, wie Diethylenglykol, Triethylenglykol, 4,4'-Dihydroxyethoxydiphenyldimethylmethan, Hexandiol und Formaldehyd herstellbaren Verbindungen in Frage. Auch durch Polymerisation cyclischer Acetale lassen sich geeignete Polyacetale herstellen.

Als Hydroxylgruppen aufweisende Polycarbonate kommen solche der an sich bekannten Art in Betracht, die beispielsweise durch Umsetzung von Diolen, wie Propandiol-(1,3), Butandiol-(1,4) und/oder Hexandiol-(1,6), Diethylenglykol, Triethylenglykol oder Tetraethylenglykol mit Diarylcarbonaten, z. B. Diphenylcarbonat, oder Phosgen hergestellt werden können.

Die Polyhydroxylverbindungen können einzeln oder in Form von Mischungen verwendet werden. Bewährt haben sich beispielsweise auch Mischungen aus Polyester- und Polyetherolen, wobei das Verhältnis der Komponenten je nach Verwendungszweck des herzustellenden Polyurethanschaumstoffs in breiten Grenzen, beispielsweise im Gewichtsverhältnis von Polyesterol zu Polyetherol von 20 : 80 bis 80 : 20, variiert werden kann.

Gegebenenfalls kann es zweckmäßig sein, neben den genannten Polyhydroxylverbindungen zusätzlich Kettenverlängerungsmittel zur Herstellung der Polyurethanschaumstoffe mitzuverwenden. Als derartige Mittel kommen polyfunktionelle, insbesondere di- und trifunktionelle Verbindungen mit Molekulargewichten von 17 bis 600, vorzugsweise 60 bis 300, in Betracht. Verwendet werden beispielsweise Di- und Trialkanolamine, wie Diethanolamin und Triethanolamin, aliphatische und aromatische Diamine, wie z. B. Ethylendiamin, Butylendiamin-1,4, Hexamethylendiamin-1,6, 4,4'-Diamino-diphenylmethan, 3,3'-dialkylsubstituierte 4,4'-Diamino-diphenylmethane, 2,4- und 2,6-Toluylendiamin und vorzugsweise aliphatische Diole und Triole mit 2 bis 6 Kohlenstoffatomen, wie Ethylenglykol, 1,4-Butylenglykol, 1,6-Hexamethylenglykol, Glyzerin und Trimethylolpropan.

Sofern Kettenverlängerungsmittel eingesetzt werden, kommen diese in Mengen von 0,05 bis 10 Gew.-Teilen, vorzugsweise von 0,1 bis 3 Gew.-Teilen pro 100 Gew.-Teile Polyhydroxylverbindungen zur Anwendung.

Zu Treibmitteln, welche zur Herstellung der Polyurethan-Weichschaumstoffe verwendet werden können, gehört vorzugsweise Wasser, das mit Isocyanatgruppen unter Bildung von Kohlendioxid reagiert. Die Wassermengen, die zweckmäßigerweise eingesetzt werden, betragen 0,1 bis 8 Gew.-Teile, vorzugsweise 1,5 bis 5 Gew.-Teile, bezogen auf 100 Gew.-Teile Polyhydroxylverbindung.

Im Gemisch mit Wasser können auch physikalisch wirkende Treibmittel eingesetzt werden. Geeignet sind Flüssigkeiten, welche gegenüber den erfindungsgemäßen Polyisocyanatmischungen inert sind und Siedepunkte unter 100°C, vorzugsweise unter 50°C, insbesondere zwischen -50°C und 30°C bei Atmosphärendruck aufweisen, so daß sie unter dem Einfluß der exothermen Polyadditionsreaktion verdampfen. Beispiele derartiger, vorzugsweise verwendbarer Flüssigkeiten sind Kohlenwasserstoffe, wie Pentan, n- und iso-Butan und Propan, Ether, wie Dimethylether und Diethylether, Ketone, wie Aceton und Methylethylketon, Ethylacetat und vorzugsweise halogenierte Kohlenwasserstoffe, wie Methylenchlorid, Trichlorfluormethan, Dichlordifluormethan, Dichlormonofluormethan, Dichlortetrafluorethan und 1,1,2-Trichlor-1,2,2-trifluorethan. Auch Gemische dieser niedrigsiedenden Flüssigkeiten untereinander und/oder mit anderen substituierten oder unsubstituierten Kohlenwasserstoffen können verwendet werden.

Die neben Wasser erforderliche Menge an physikalisch wirkenden Treibmitteln kann in Abhängigkeit von der

gewünschten Schaumstoffdichte auf einfache Weise ermittelt werden und beträgt ungefähr 0 bis 50 Gewichtsteile, vorzugsweise 0 bis 20 Gewicht steile pro 100 Gewichtsteile Polyhydroxylverbindung. Gegebenenfalls kann es zweckmäßig sein, die erfindungsgemäße Polyisocyanatmischung mit dem physikalisch wirkenden Treibmittel zu mischen und dadurch die Viskosität zu verringern.

Zur Beschleunigung der Umsetzung zwischen den Polyhydroxylverbindungen, Wasser und gegebenenfalls Kettenverlängerungsmittel und den erfindungsgemäßen Polyisocyanatmischungen auf Roh-MDI-basis werden der Reaktionsmischung übliche Polyurethankatalysatoren einverleibt. Vorzugsweise verwendet werden basische Polyurethankatalysatoren, beispielsweise tertiäre Amine, wie Dimethylbenzylamin, Dicyclohexylmethylamin, Dimethylcyclohexylamin, N,N,N',N'-Tetramethyl-diamino-diethylether, Bis-(dimethylaminopropyl)-harnstoff, N-Methyl- bzw. N-Ethylmorpholin, Dimethylpiperazin, Pyridin, 1,2-Dimethylimidazol, 1-Azabicyclo-(3,3,0)-octan, Dimethylaminoethanol, 2-(N,N-Dimethylaminoethoxy)ethanol, N,N',N''-Tris-(dialkylaminoalkyl)-hexahydrotriazine, z. B. N,N',N''-Tris-(dimethylaminopropyl)-s-hexahydrotriazin und insbesondere Triethylendiamin. Geeignet sind jedoch auch Metallsalze, wie Eisen-(II)-chlorid, Zinkchlorid, Bleioctoat und vorzugsweise Zinnsalze, wie Zinndioctoat, Zinndiethylhexoat und Dibutylzinndilaurat sowie insbesondere Mischungen aus tertiären Aminen und organischen Zinnsalzen. Eingesetzt werden zweckmäßigerweise 0,1 bis 10 Gew.-%, vorzugsweise 0,3 bis 3 Gew.-% Katalysator auf Basis tertiärer Amine und/oder 0,01 bis 0,5 Gew.-%, vorzugsweise 0,03 bis 0,25 Gew.-% Metallsalze, bezogen auf das Gewicht an Polyhydroxylverbindungen.

Der Reaktionsmischung können auch noch Hilfsmittel und/oder Zusatzstoffe einverleibt werden. Genannt seien beispielsweise Stabilisatoren, Hydrolyseschutzmittel, Porenregler, fungistatisch und bakteriostatisch wirkende Substanzen, Farbstoffe, Pigmente, Füllstoffe, oberflächenaktive Stoffe und Flammschutzmittel.

In Betracht kommen beispielsweise oberflächenaktive Substanzen, welche zur Unterstützung der Homogenisierung der Ausgangsstoffe dienen und gegebenenfalls auch geeignet sind, die Zellstruktur der Schaumstoffe zu regulieren. Genannt seien beispielhaft Siloxan-Oxyalkylen-Mischpolymerisate und andere Organopolysiloxane, oxethylierte Alkylphenole, oxethylierte Fettalkohole, Paraffinöle, Rizinusöl- bzw. Rizinolsäureester und Türkischrotöl, die in Mengen von 0,05 bis 5, vorzugsweise von 0,1 bis 2 Gewichtsteilen pro 100 Gewichtsteilen Polyhydroxylverbindung angewandt werden.

Als Flammschutzmittel sind beispielsweise Phosphor- und/ oder Halogenatome enthaltende Verbindungen, wie z. B. Trikresylphosphat, Tris-2-chlorethylphosphat, Tris-chlorpropylphosphat und Tris-2,3-dibrompropylphosphat geeignet.

Außer den bereits genannten halogensubstituierten Phosphaten können auch anorganische Flammschutzmittel, beispielsweise Antimontrioxid, Arsenoxid, Ammoniumphosphat und Calciumsulfat, oder Melamin zum Flammfestmachen der Polyurethan-Weichschaumstoffe verwendet werden.

Im allgemeinen hat es sich als vorteilhaft erwiesen, 5 bis 50 Gewichtsteile, vorzugsweise 5 bis 25 Gewichtsteile der genannten Flammschutzmittel pro 100 Gewichtsteile Polyhydroxylverbindung zu verwenden.

Zur Herstellung der Polyurethan-Weichschaumstoffe werden die erfindungsgemäßen Polyisocyanatmischungen, Polyhydroxylverbindungen und gegebenenfalls Kettenverlängerungsmittel in Gegenwart von Katalysatoren, Treibmitteln und gegebenenfalls Hilfsmitteln und/oder Zusatzstoffen bei Temperaturen von 0 bis 70° C, vorzugsweise 15 bis 50° C in solchen Mengenverhältnissen zur Reaktion gebracht, daß pro NCO-Gruppe 0,5 bis 2, vorzugsweise 0,8 bis 1,3 und insbesondere ungefähr ein reaktive(s) Wasserstoffatom(e) gebunden an die Polyhydroxylverbindung und gegebenenfalls Kettenverlängerungsmittel vorliegen und das Molverhältnis von Äquivalente Wasser zu Äquivalente NCO-Gruppen 0,5 bis 5 : 1, vorzugsweise 0,7 bis 0,95 : 1 und insbesondere 0,75 bis 0,85 : 1 beträgt.

Die Polyurethan-Weichschaumstoffe werden nach dem one shot-Verfahren hergestellt, wobei die Ausgangskomponenten, Hilfsmittel und Zusatzstoffe bei Verwendung einer Mischkammer mit mehreren Zulaufdüsen einzeln zugeführt und in der Mischkammer intensiv vermischt werden. Als besonders zweckmäßig hat es sich jedoch erwiesen, so daß diese Verfahrensweise bevorzugt angewandt wird, nach dem Zweikomponenten-Verfahren zu arbeiten und die Polyhydroxylverbindung, Katalysatoren, Treibmittel und gegebenenfalls Kettenverlängerungsmittel, Hilfsmittel und/oder Zusatzstoffe zu der sogenannten A-Komponente zu vereinigen und als B-Komponente die erfindungsgemäße Polyisocyanatmischung gegebenenfalls im Gemisch mit physikalisch wirkenden Treibmitteln, Hilfsmittel und/oder Zusatzstoffen zu verwenden. Vorteilhaft ist hierbei, daß die A- und B-Komponente raumsparend transportiert und beschränkte Zeit gelagert werden können und vor Herstellung der Polyurethan-Weichschaumstoffe nur noch intensiv gemischt werden müssen. Die Reaktionsmischungen können in offenen oder geschlossenen Formwerkzeugen verschäumt werden.

Die aus den flüssigen, Harnstoffgruppen enthaltenden Polyisocyanatmischungen hergestellten Polyurethan-Weichschaumstoffe besitzen Dichten von 10 bis 150 kg/m³, vorzugsweise 20 bis 70 kg/m³ und zeichnen sich durch eine erhöhte mechanische Beständigkeit bei Beanspruchung unter erhöhter Temperatur und Luftfeuchtigkeit aus.

Die in den Beispielen genannten Teile beziehen sich auf das Gewicht.

**Beispiel 1**

Herstellung der Harnstoffgruppen enthaltenden Polyisocyanatmischung

Zu 100 Teilen einer Mischung aus 4,4'- und 2,4'-MDI im Gewichtsverhältnis 75 : 25 wurden bei 80° C unter starkem Rühren (1200 Upm) 42,3 eines Blockcopolyoxyalkylen-polyamins mit einer Aminzahl von 27 und einer Hydroxylzahl von 0, das hergestellt wurde durch Aminierung eines Polyether-diols auf Basis von Dipropylenglykol/1,2-Propylenoxid/Ethylenoxid, innerhalb von 30 Minuten hinzugefügt.

Nach einer Reaktionszeit von 15 Minuten ließ man die Reaktionsmischung auf 40° C abkühlen und verdünnte sie im Gewichtsverhältnis 80 : 20 mit Roh-MDI mit einem MDI-Gehalt von 47 Gew.-%. Es entstand eine dunkelbraune, flüssige, homogene Polyisocyanatmischung mit einem NCO-Gehalt von 24,5 Gew.-%.

**Vergleichsbeispiel A**

Herstellung einer Urethangruppen enthaltenden Polyisocyanatmischung

Man verführ analog den Angaben von Beispiel 1, verwandte jedoch zur Modifizierung anstelle des Blockcopolyoxyalkylen-polyamins das diesem zugrundeliegende Dipropylen-polyoxypropylen-polyoxyethylenglykol.

Es entstand eine dunkelbraune, homogene Flüssigkeit mit einem NCO-Gehalt von 24,6 Gew.-%.

**Beispiel 2**

A-Komponente:
Mischung aus
90,35 Teilen eines mit Glycerin gestarteten Polyoxypropylen-polyoxyethylen-triols mit 20 Gew.-% endständigen Polyoxyethylengruppen und einer Hydroxylzahl von 26,
2,8 Teilen Wasser,
0,35 Teilen einer 33 gew.-%-igen Lösung von Triethylendiamin in Dipropylenglykol,
0,2 Teilen Bis-(N,N-dimethylaminoethyl)-ether,
0,2 Teilen N,N-Dimethylaminoethanol und
6 Teilen Trichlorfluormethan.

B-Komponente:
58,5 Gew.-Teile der Polyisocyanatmischung nach Beispiel 1.

Die A- und B-Komponenten wurden bei 23°C 8 Sekunden intensiv gemischt. 880 g der reaktionsfähigen Mischung wurden in eine auf 50°C erwärmte Metallform mit den inneren Abmessungen 40 x 40 x 10 cm eingefüllt und in dem geschlossenen Formwerkzeug aufschäumen gelassen.

Es entstand ein elastischer Formkörper mit einer Dichte von 51 g/l.

**Vergleichsbeispiel B**

A-Komponente: analog Beispiel 2
B-Komponente: 58,3 Gew.-Teile der Polyisocyanatmischung nach Vergleichsbeispiel A.
>r Die Herstellung des Formteils erfolgte analog den Angaben des Beispiels 2.

Die Formteile nach Beispiel 2 und dem Vergleichsbeispiel B ergaben vergleichbare Meßwerte für die Reißdehnung, Zugfestigkeit, den Druckverformungsrest und die Härte.

Zur Bestimmung des mechanischen Verhaltens nach der Alterung bei erhöhter Temperatur und Luftfeuchtigkeit wurden die Formteile folgenden Testmethoden unterworfen:

Alterung im Autoklav:
5 Stunden bei 120° C unter gesättigtem Wasserdampf und anschließend
24 Stunden bei 70° C im Trockenschrank unter Frischluftzufuhr.

Hitzealterung:
22 Stunden bei 140° C im Trockenschrank

Die prozentuale Abnahme des Druckverformungsrest nach DIN 53 572, der Stauchhärte nach DIN 53 577 und

der Zugfestigkeit nach DIN 53 571 wurde in der folgenden Tabelle zusammengefaßt.

| | Beispiel 2 | Vergleichsbeispiel B |
|---|---|---|
| Autoklavalterung: | | |
| Druckverformungsrest | 2 % | 29 % |
| Stauchhärte | 36 % | 45 % |
| Hitzealterung: | | |
| Zugfestigkeit | 7 % | 19 % |

**Patentansprüche**

1. Bei Raumtemperatur flüssige, Harnstoffgruppen und Oxyalkyleneinheiten gebunden enthaltende Polyisocyanatsemiprepolymere mit einem NCO-Gehalt von 15 bis 30 Gew.-% und einem Gehalt an gegebenenfalls modifizierten Diphenylmethan-diisocyanat-Isomeren von 55 bis 90 Gew.-%, erhalten durch partielle Umsetzung von

A) Polyoxyalkylen-polyaminen mit einer Funktionalität von 2 bis 5 und einer Aminzahl von 20 bis 250 mit

B1) einer Mischung aus Diphenylmethan-diisocyanaten und Polyphenyl-polymethylen-polyisocyanaten mit einem Diphenylmethan-diisocyanat-Gehalt von 55 bis 90 Gew.-% oder

B2) mindestens einem Diphenylmethan-diisocyanat-Isomeren und anschließendem Verdünnen des Harnstoffgruppen und Oxyalkyleneinheiten gebunden enthaltenden Reaktionsproduktes mit einer Mischung aus Diphenylmethan-diisocyanaten und Polyphenyl-polymethylen-polyisocyanaten mit einem Diphenylmethan-diisocyanatgehalt von 30 bis 85 Gew.-%.

2. Bei Raumtemperatur flüssige, Harnstoffgruppen und Oxyalkyleneinheiten gebunden enthaltende Diphenylmethan-diisocyanatsemiprepolymere mit einem NCO-Gehalt von 15 bis 28 Gew.-%, erhalten durch partielle Umsetzung von

A) Polyoxyalkylen-polyaminen mit einer Funktionalität von 2 bis 5 und einer Aminzahl von 20 bis 250 mit

B3) einer Diphenylmethan-diisocyanat-Mischung aus
40 bis 98 Gew.-% 4,4'-Diphenylmethandiisocyanat,
60 bis 2 Gew.-% 2,4'-Diphenylmethandiisocyanat und
0 bis 2 Gew.-% 2,2'-Diphenylmethandiisocyanat,

wobei die Gew.-% bezogen sind auf das Gesamtgewicht der Diphenylmethandiisocyanat-Mischung.

3. Bei Raumtemperatur flüssige, Harnstoffgruppen enthaltende Polyisocyanatmischungen nach Anspruch 1, dadurch gekennzeichnet, daß man die

A) Polyoxyalkylen-polyamine und

B1) Mischungen aus Diphenylmethan-diisocyanaten und Polyphenyl-polymethylen-polyisocyanaten oder

B2) Diphenylmethan-diisocyanat-Isomeren

in solchen Mengen zur Reaktion bringt, daß das NCO : $NH_2$-Verhältnis 1 : 0,005 bis 1 : 0,35 beträgt.

4. Bei Raumtemperatur flüssige, Harnstoffgruppen enthaltende Polyisocyanatmischungen nach Anspruch 1, dadurch gekennzeichnet, daß die Polyoxyalkylen-polyamine (A) Strukturen der allgemeinen Formeln

$$H_2N-(R^2O)_x-R^1-NH_2 \qquad (I),$$

$$H_2N-R^1-(OR^2)_x-NH-(R^2O)_y-R^1-NH_2 \qquad (II),$$

$$H_2N-R^1-(OR^2)_x-T\begin{matrix} / (R^2O)_y-R^1-NH_2 \\ \backslash (R^2O)_z-R^1-NH_2 \end{matrix} \qquad (III)$$

und/oder

$$\begin{array}{l} H_2N\text{-}R^1\text{-}(OR^2)_y \diagdown \\ \qquad T\text{-}(R^2O)_x\text{-}R^1\text{-}NH\text{-}R^1\text{-}(OR^2)_{x'}\text{-}T \\ H_2N\text{-}R^1\text{-}(OR^2)_z \diagup \end{array} \begin{array}{l} \diagup (R^2O)_{y'}\text{-}R^1\text{-}NH_2 \\ \\ \diagdown (R^2O)_{z'}\text{-}R^1\text{-}NH_2 \end{array} \qquad (IV)$$

besitzen, in denen bedeuten:

$R^1$ und $R^2$ gleiche oder verschiedene, gegebenenfalls substituierte Alkylenreste mit 2 bis 10 Kohlenstoffatomen, wobei die Alkylenreste $R^2$ der Alkoxygruppen ebenfalls gleich oder verschieden sein können,

T ein keine reaktiven Wasserstoffatome enthaltender Rest eines trifunktionellen Startermoleküls für die Alkylenoxidpolymerisation und

x, x', y, y', z und z' gleiche oder verschiedene ganze Zahlen größer als null, so daß die Summe eine Aminzahl von 20 bis 250 ergibt.

5. Verfahren zur Herstellung vonbei Raumtemperatur flüssigen, Harnstoffgruppen und Oxyalkyleneinheiten gebunden enthaltenden Polyisocyanatsemiprepolymeren mit einem NCO-Gehalt von 15 bis 30 Gew.-% und einem Gehalt an ggf. modifizierten Diphenylmethan-diisocyanat-Isomeren von 55 bis 90 Gew.-%, <u>dadurch gekennzeichnet</u>, daß man

A) Polyoxyalkylen-polyamine mit einer Funktionalität von 2 bis 5 und einer Aminzahl von 20 bis 250 und

B1) eine Mischung aus Diphenylmethan-diisocyanaten und Polyphenyl-polymethylen-polyisocyanaten mit einem Diphenylmethan-diisocyanat-Gehalt von 55 bis 90 Gew.-% oder

B2) mindestens ein Diphenylmethan-diisocyanat-Isomeres in Äquivalenzverhältnissen von NCO- : $NH_2$-Gruppen von 1 : 0,005 bis 0,35 bei Temperaturen von 10 bis 120°C zur Reaktion bringt und danach die nach B2) erhaltenen harnstoffgruppenhaltigen Reaktionsprodukte mit einer Mischung aus Diphenylmethan-diisocyanaten und Polyphenyl-polymethylen-polyisocyanaten mit einem Diphenylmethan-diisocyanat-Gehalt von 30 bis 85 Gew.-% verdünnt.

6. Verfahren nach Anspruch 5, <u>dadurch gekennzeichnet</u>, daß die Polyoxyalkylen-polyamine (A) Strukturen der allgemeinen Formeln

$$H_2N\text{-}(R^2O)_x\text{-}R^1\text{-}NH_2 \qquad (I),$$

$$H_2N\text{-}R^1\text{-}(OR^2)_x\text{-}NH\text{-}(R^2O)_y\text{-}R^1\text{-}NH_2 \qquad (II),$$

$$H_2N\text{-}R^1\text{-}(OR^2)_x\text{-}T \begin{array}{l} \diagup (R^2O)_y\text{-}R^1\text{-}NH_2 \\ \\ \diagdown (R^2O)_z\text{-}R^1\text{-}NH_2 \end{array} \qquad (III)$$

und/oder

$$\begin{array}{l} H_2N\text{-}R^1\text{-}(OR^2)_y \diagdown \\ \qquad T\text{-}(R^2O)_x\text{-}R^1\text{-}NH\text{-}R^1\text{-}(OR^2)_{x'}\text{-}T \\ H_2N\text{-}R^1\text{-}(OR^2)_z \diagup \end{array} \begin{array}{l} \diagup (R^2O)_{y'}\text{-}R^1\text{-}NH_2 \\ \\ \diagdown (R^2O)_{z'}\text{-}R^1\text{-}NH_2 \end{array} \qquad (IV)$$

besitzen, in denen bedeuten:

$R^1$ und $R^2$ gleiche oder verschiedene, gegebenenfalls substituierte Alkylenreste mit 2 bis 10 Kohlenstoffatomen, wobei die Alkylenreste $R^2$ der Alkoxygruppen ebenfalls gleich oder verschieden sind,

T ein keine reaktiven Wasserstoffatome enthaltender Rest eines trifunktionellen Startermoleküls für die Alkylenoxid-polymerisation und

x, x', y, y', z und z' gleiche oder verschiedene ganze Zahlen größer als null.

7. Verfahren nach Anspruch 5, <u>dadurch gekennzeichnet</u>, daß man die Umsetzung unter starkem Rühren mit Rührerdrehzahlen von 200 bis 1500 pro Minute durchführt.

8. Verwendung der flüssigen, Harnstoffgruppen und Oxyalkyleinheiten gebunden enthaltenden Polyisocyanatsemiprepolymeren nach Anspruch 1 zur Herstellung von kompakten oder zelligen Polyurethan- und/oder Polyisocyanurat-Kunststoffen.

9. Verwendung der flüssigen, Harnstoffgruppen und Oxyalkyleinheiten gebunden enthaltenden Diphenylmethan-diisocyanatsemiprepolymeren nach Anspruch 2 zur Modifizierung von organischen Polyisocyanaten oder zur Herstellung von kompakten oder zelligen Polyurethan- und/oder Polyisocyanat-Kunststoffen.

10. Verfahren zur Herstellung von Polyurethan-Weichschaumstoffen durch Umsetzung von organischen Polyisocyanaten mit Polyhydroxylverbindungen und gegebenenfalls Kettenverlängerungsmitteln in Gegenwart von Katalysatoren und Treibmitteln sowie gegebenenfalls Hilfsmitteln und/oder Zusatzstoffen in geschlossenen oder offenen Formwerkzeugen, dadurch gekennzeichnet, daß man als organische Polyisocyanate bei Raumtemperatur flüssige, Harnstoffgruppen und Oxyalkyleinheiten gebunden enthaltende Polyisocyanatsemiprepolymeren nach Anspruch 1 verwendet.

## Claims

1. A polyisocyanate semiprepolymer wuhich is liquid at room temperature, contains urea groups and oxyalkylene units, has an NCO content of from 15 to 30 % by weight and a content of unmodified or modified diphenylmethane diisocyanate isomers of from 55 to 90 % by weight and is obtained by partial reaction of

A) polyoxyalkylenepolyamines having a functionality of from 2 to 5 and an amine number of from 20 to 250 with

B1) a mixture of diphenylmethane diisocyanates and polyphenylpolymethylene polyisocyanates having a diphenylmethane diisocyanate content of from 55 to 90 % by weight or

B2) one or more diphenylmethane diisocyanate isomers, followed by dilution of the reaction product containing urea groups and oxyalkylene units with a mixture of diphenylmethane diisocyanates and polyphenylpolymethylene polyisocyanates having a diphenylmethane diisocyanate content of from 30 to 85 % by weight.

2. A diphenylmethane diisocyanate semiprepolymer which is liquid at room temperature, contains urea groups and oxyalkylene units, has an NCO content of from 15 to 28 % by weight and is obtained by partial reaction of

A) polyoxyalkylenepolyamines having a functionality of from 2 to 5 and an amine number of from 20 to 250 with

B3) a diphenylmethane diisocyanate mixture of
from 40 to 98 % by weight of 4,4'-diphenylmethane diisocyanate,
from 60 to 2 % by weight of 2,4'-diphenylmethane diisocyanate and
from 0 to 2 % by weight of 2,2'-diphenylmethane diisocyanate,
the percentages by weight being based on the total weight of the diphenylmethane diisocyanate mixture.

3. A polyisocyanate mixture as claimed in claim 1 which is liquid at room temperature and contains urea groups, wherein

A) polyoxyalkylenepolyamines and

B1) a mixture of diphenylmethane diisocyanates and polyphenylpolymethylene polyisocyanates or

B2) diphenylmethane diisocyanate isomers

are reacted in amounts such that the NCO : $NH_2$ ratio is from 1 : 0.005 to 1 : 0.35.

4. A polyisocyanate mixture as claimed in claim 1 which is liquid at room temperature and contains urea groups, wherein polyoxyalkylenepolyamines (A) have structures of the formulae

$$H_2N-(R^2O)_x-R^1-NH_2 \qquad (I),$$

$$H_2N-R^1-(OR^2)_x-NH-(R^2O)_y-R^1-NH_2 \qquad (II),$$

$$H_2N-R^1-(OR^2)_x-T\begin{array}{l} /\,(R^2O)_y-R^1-NH_2 \\ \backslash\,(R^2O)_z-R^1-NH_2 \end{array} \qquad (III)$$

and/or

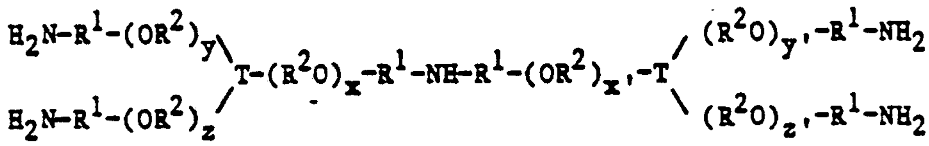 (IV)

where

$R^1$ and $R^2$ are identical or different, unsubstituted or substituted alkylene radicals of 2 to 10 carbon atoms, and the alkylene radicals $R^2$ of the alkoxy groups may likewise be identical or different,

T is a radical of a trifunctional initiator molecule for the alkylene oxide polymerization, the said radical containing no reactive hydrogen atoms, and

x, x', y, y', z and z' are identical or different integers greater than zero, so that the sum gives an amine number of from 20 to 250.

5. A process for the preparation of a polyisocyanate semiprepolymer which is liquid at room temperature, contains urea groups and oxyalkylene units and has an NCO content of from 15 to 30 % by weight and a content of unmodified or modified diphenylmethane diisocyanate isomers of from 55 to 90 % by weight, wherein

A) polyoxyalkylenepolyamines having a functionality of from 2 to 5 and an amine number of from 20 to 250 and

B1) a mixture of diphenylmethane diisocyanates and polyphenylpolymethylene polyisocyanates having a diphenylmethane diisocyanate content of from 55 to 90 % by weight or

B2) one or more diphenylmethane diisocyanate isomers are reacted at from 10 to 120°C in amounts such that the ratio of the number of NCO groups to the number of $NH_2$ groups is from 1 : 0.005 to 1 : 0.35, and thereafter the reaction products containing urea groups and obtained according to B2) are diluted with a mixture of diphenylmethane diisocyanates and polyphenylpolymethylene polyisocyanates having a diphenylmethane diisocyanate content of from 30 to 85 % by weight.

6. A process as claimed in claim 5, wherein the polyoxyalkylenepolyamines (A) have structures of the formulae

$$H_2N-(R^2O)_x-R^1-NH_2 \qquad (I),$$

$$H_2N-R^1-(OR^2)_x-NH-(R^2O)_y-R^1-NH_2 \qquad (II),$$

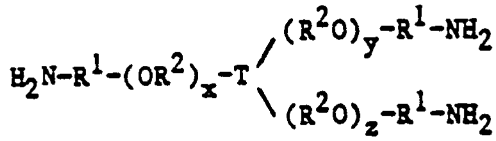 (III)

and/or

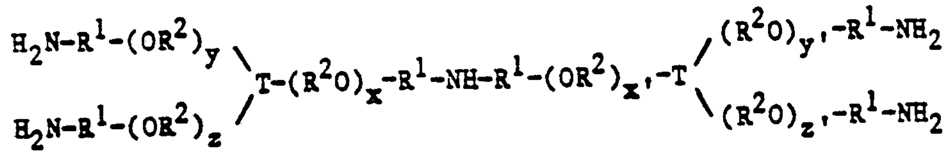 (IV)

where $R^1$ and $R^2$ are identical or different, and unsubstituted or substituted alkylene radicals of 2 to 10 carbon atoms, and the alkylene radicals $R^2$ of the alkoxy groups are likewise identical or different, T is a radical of a trifunctional initiator molecule for the alkylene oxide polymerization, the said radical containing no reactive hydrogen atoms, and x, x', y, y', z and z' are identical or different integers greater than zero.

7. A process as claimed in claim 5, wherein the reaction is carried out with vigorous stirring at stirrer speeds of from 200 to 1,500 r.p.m.

8. Use of a liquid polyisocyanate semiprepolymer as claimed in claim 1, which contains urea groups and oxyalkylene units, for the preparation of compact or cellular polyurethane and/or polyisocyanate plastics.

9. Use of a liquid diphenylmethane diisocyanate semiprepolymer as claimed in claim 2, which contains urea groups and oxyalkylene units, for the modification of organic polyisocyanates or for the preparation of compact or cellular polyurethane and/or polyisocyanate plastics.

10. A process for the preparation of a soft polyurethane foam by reacting organic polyisocyanates with polyhydroxy compounds and, if required, chain extenders in the presence of catalysts and blowing agents and, if required, assistants and/or additives in a closed or open mold, wherein the organic

polyisocyanates used are polyisocyanate semiprepolymers as claimed in claim 1 which are liquid at room temperature and contain urea groups and oxyalkylene units.

**Revendications**

1. Semi-prépolymères liquides à la température ambiante, contenant en liaison des groupes urée et des motifs oxyalkylène, ayant une teneur en NCO de 15 à 30 % en poids et une teneur en isomères de diisocyanate de diphénylméthane éventuellement modifiés de 55 à 90 % en poids, obtenus par réaction partielle de

A) polyoxyalkylène-polyamines ayant une fonctionnalité de 2 à 5 et un indice d'amine de 20 à 250 avec

B1) un mélange de diisocyanates de diphenylméthane et de polyisocyanates de polyphényl-polyméthylène ayant une teneur en diisocyanates de diphénylméthane de 55 à 90 % en poids, ou

B2) au moins un isomère de diisocyanate de diphénylméthane, suivie de dilution du produit de réaction, contenant en liaison des groupes urée et des motifs oxyalkylène, par un mélange de diisocyanates de diphénylméthane et de polyisocyanates de polyphényl-polyméthylène ayant une teneur en diisocyanates de diphénylméthane de 30 à 85 % en poids.

2. Semi-prépolymères liquides à la température ambiante, contenant en liaison des groupes urée et des motifs oxyalkylène, ayant une teneur en NCO de 15 à 28 % en poids, obtenus par réaction partielle de

A) polyoxyalkylène-polyamines ayant une fonctionnalité de 2 à 5 et un indice d'amine de 20 à 250 avec

B3) un mélange de diisocyanates de diphénylméthane se composant de
40 à 98 % en poids de diisocyanate de 4,4'-diphénylméthane
60 à 2 % en poids de diisocyanate de 2,4'-diphénylméthane et
0 à 2 % en poids de diisocyanate de 2,2'-diphénylméthane les pourcentages en poids étant rapportés au poids total du mélange de diisocyanates de diphénylméthane.

3. Mélanges de polyisocyanates liquides à la température ambiante, contenant des groupes urée, selon la revendication 1, caractérisés en ce qu'on met en réaction

A) les polyoxyalkylène-polyamines et

B1) les mélanges de diisocyanates de diphénylméthane et de polyisocyanates de polyphényl-polyméthylène ou

B2) les isomères de diisocyanate de diphénylméthane dans des quantités telles que le rapport NCO : $NH_2$ soit compris entre 1 : 0,005 et 1 : 0,35.

4. Mélanges de polyisocyanates liquides à la température ambiante, contenant des groupes urée, selon la revendication 1, caractérisés en ce que les polyoxyalkylène-polyamines (A) présentent des structures de formules générales

$$H_2N-(R^2O)_x-R^1-NH_2 \qquad (I)$$

$$H_2N-R^1-(OR^2)_x-NH-(R^2O)_y-R^1-NH_2 \qquad (II)$$

$$H_2N-R^1-(OR^2)_x-T\begin{cases}(R^2O)_y-R^1-NH_2\\(R^2O)_z-R^1-NH_2\end{cases} \qquad (III)$$

et/ou

$$\begin{matrix}H_2N-R^1-(OR^2)_y\\H_2N-R^1-(OR^2)_z\end{matrix}\!\!>T-(R^2O)_x-R^1-NH-R^1-(OR^2)_{x'}-T\!<\!\!\begin{matrix}(R^2O)_{y'}-R^1-NH_2\\(R^2O)_{z'}-R^1-NH_2\end{matrix} \qquad (IV)$$

dans lesquelles

$R^1$ et $R^2$ peuvent être identiques ou différents et représentent des radicaux alkylène à 2 - 10 atomes de carbone éventuellement substitues, les radicaux alkylène $R^2$ des groupes alcoxy pouvant être également identiques ou différents,

T représente un radical, ne contenant pas d'atome d'hydrogène réactif, d'une molécule trifonctionnelle de démarrage pour la polymérisation d'oxyde d'alkylène et

x, x', y, y', z et z' sont des nombres entiers supérieurs à 0, identiques ou différents, tels que leur somme donne un indice d'amine de 20 à 250.

5. Procédé de préparation de semi-prépolymères de polyisocyanates liquides à la température

ambiante, contenant en liaison des groupes urée et des motifs oxyalstylène, ayant une teneur en NCO de 15 à 30 % en poids et une teneur en isomères de diisocyanate de diphénylméthane éventuellement modifiés de 55 à 90 % en poids, caractérisé en ce qu'on met en réaction, à des températures de 10 à 120°C,

A) des polyoxyalkylène-polyamines ayant une fonctionnalité de 2 à 5 et un indice d'amine de 20 à 250 et

B1) un mélange de diisocyanates de diphénylméthane et de polyisocyanates de polyphénylpolyméthylène ayant une teneur en diisocyanates de diphénylméthane de 55 à 90 % en poids ou

B2) au moins un isomère de diisocyanate de diphénylméthane,

dans des rapports d'équivalence des groupes NCO : $NH_2$ de 1 : 0,005 à 0,35, puis on dilue les produits de réaction contenant des groupes urée, obtenus suivant B2), avec un mélange de diisocyanates de diphénylméthane et de polyisocyanates de polyphényl-polyméthylène ayant une teneur en diisocyanates de diphénylméthane de 30 à 85 % en poids.

6. Procédé selon la revendication 5, caractérisé en ce que les polyoxyalkylène-polyamines (A) présentent des structures de formules générales

$$H_2N-R^1-(OR^2)_x-NH-(R^2O)_y-R^1-NH_2 \qquad (II)$$

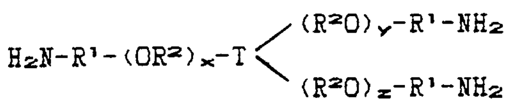

(III)

et/ou

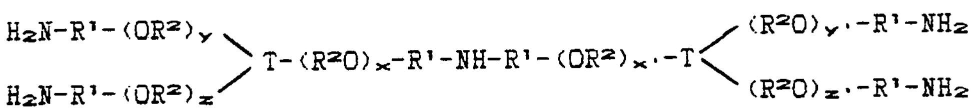

(IV)

dans lesquelles

$R^1$ et $R^2$ peuvent être identiques ou différents et représentent des radicaux alkylène à 2 - 10 atomes de carbone éventuellement substitués, les radicaux alkylène $R^2$ des groupes alcoxy pouvant être également identiques ou différents,

T représente un radical, ne contenant pas d'atome d'hydrogène réactif, d'une molécule trifonctionnelle de démarrage de la polymérisation d'oxyde d'alkylène et

x, x', y, y', z et z' sont des nombres entiers supérieurs à 0, identiques ou différents.

7. Procédé selon la revendication 5, caractérisé en ce qu'on conduit la réaction sous agitation énergique, à des vitesses de rotation de l'agitateur de 200 à 1500 tr/mn.

8. Utilisation des semi-prépolymères liquides de polyisocyanates, contenant en liaison des groupes urée et des motifs oxyalkylène selon la revendication 1, pour la fabrication de matières plastiques de polyuréthane et/ou de polyisocyanate compactes ou cellulaires.

9. Utilisation des semi-prépolymères liquides de diisocyanates de diphénylméthane, contenant en liaison des groupes urée et des motifs oxyalkylène selon la revendication 2, pour la modification de polyisocyanates organiques ou pour la fabrication de matières plastiques de polyuréthane et/ou de polyisocyanate compactes ou cellulaires.

10. Procédé de fabrication de mousses molles de polyuréthane par réaction de polyisocyanates organiques avec des composés polyhydroxylés et éventuellement avec des agents d'allongement de chaîne en présence de catalyseurs, d'agents moussants et, le cas échéant, d'adjuvants et/ou d'additifs dans des outils de moulage fermes ou ouverts, caractérisé en ce qu'on utilise, comme polyisocyanates organiques, des semi-prépolymères de polyisocyanates, contenant en liaison des groupes urée et des motifs oxyalkylène, liquides à la température ambiante selon la revendication 1.